# EUROPÄISCHE PATENTSCHRIFT

(11) **EP 1 108 439 B1**
(45) Veröffentlichungstag und Bekanntmachung des Hinweises auf die Patenterteilung: **15.06.2005**
(21) Anmeldenummer: 00710042.3
(22) Anmeldetag: 15.12.2000
(51) Int. Cl.: A61M 15/08, B05B 11/00, A61M 15/00

(54) **Spender für Medien**
Product dispenser
Distributeur de produit

(30) Priorität: 15.12.1999 DE 19960459
(43) Veröffentlichungstag der Anmeldung: 20.06.2001
(73) Patentinhaber: Ing. Erich Pfeiffer GmbH, 78315 Radolfzell (DE)
(72) Erfinder: Ritsche, Stefan, 78315 Radolfzell (DE); Greiner-Perth, Jürgen, 78244 Gottmadingen (DE)
(74) Vertreter: Patentanwälte Ruff, Wilhelm, Beier, Dauster & Partner

(56) Entgegenhaltungen:
- EP-A- 0 546 607
- US-A- 3 884 229
- US-A- 3 906 950
- US-A- 4 131 217
- US-A- 5 503 302

## Beschreibung

Die Erfindung betrifft einen Spender, der zum Austrag oder zur Lagerung fester Medien, insbesondere aber mindestens eines fließfähigen Mediums geeignet sein soll. Jedes dieser Medien kann flüssig, pastös, pulver- und/oder gasförmig sein.

Bei medizinischen Spritzen für Injektionen oder dgl. besteht der Spender aus einem Zylinder mit Kolben und einer Injektions-Nadel, die sowohl als Einlaß wie auch als Auslaß für das Medium dient. Demgegenüber sind diese Durchtritte beim Erfindungsgegenstand gesondert bzw. im Abstand voneinander vorgesehen, z.B. an voneinander abgekehrten Enden eines Gehäuses, das vom Medium durchströmt wird und ein Ventil-oder ein Pumpengehäuse sein kann. Dieses Gehäuse kann drei-, vier- oder mehrfach unterschiedliche sowie gegeneinander abgesetzte Innenweiten aufweisen und mindestens zwei, drei oder mehr gesonderte Bauteile im Innern aufnehmen. Außerdem weist das Gehäuse Mittel, wie einen Flansch, zur Verbindung mit einer Basis auf, die allein ein Medienspeicher sein oder einen Medienspeicher umfassen kann.

Die US-A-5,503,302 beschreibt einen Zerstäuber, der auf einem Teil eines zweiteiligen Gehäuses angebracht ist, in dessen zweitem Teil eine Flasche mit dem auszugebenden Medium aufgenommen ist. Die Flasche ist mit einem durchstechbaren Verschluss versehen und der Zerstäuber hat als Saugrohr einen Einstechdorn. Die beiden Gehäuseteile werden zusammengeschraubt. Dabei durchsticht das Saugrohr den Verschluss und taucht in die Flasche ein.

Die US-A-3,884,229 beschreibt eine hypodermische Spritze mit je einer Nadel auf beiden Seiten, von denen die eine in einen Verschluss-Stopfen einer Ampulle einstechen kann, die in einem Gehäuse aufgenommen ist. Der Spritzenkolben greift in die Ampulle ein und setzt die Flüssigkeit in der Ampulle unter den Applikationsdruck.

Der Erfindung liegt die Aufgabe zu Grunde, einen Spender zu schaffen, bei welchem Nachteile bekannter Ausbildungen bzw. der genannten Spritzen vermieden sind. Insbesondere soll mindestens einer der Medien-Durchtritte zum Durchstechen von Membranen, Gewebe oder dgl. geeignet sein. Des weiteren sollen mit dem Spender unterschiedliche Medien zu mischen und auszutragen sein. Die Handhabung des Spenders soll einfach sein.

Diese Aufgabe wird durch den Anspruch 1 gelöst.

Das Gehäuse weist ein frei vorstehendes Stech- oder Öffnungsglied, wie eine hohle oder zugespitzte Stechnadel aus einem Werkstoff auf, welcher in seinen Eigenschaften demjenigen von Stahl oder gehärtetem Stahl entspricht. Das Stechglied kann eine Außenweite von weniger als 2 mm und eine Wandungsdicke haben, die größer als die Hälfte oder zwei Drittel der Innenweite ist. Bei Verwendung des Stechgliedes als Auslaß können mit dem Spender Injektionen durchgeführt werden. Bei Verwendung als Einlaß kann der Spender allein durch einen Stechvorgang an eine Ampulle, wie eine Carpule, abgedichtet angeschlossen werden. Das Stechglied könnte zwar gegen Federkraft bewegbar am Gehäuse gelagert sein, ist jedoch mit diesem zweckmäßig unlösbar verbunden, z.B. durch den Spritzvorgang bei der Gußfertigung des Gehäuses aus Kunststoff. Alle Teile des Spenders, ggf. eine oder mehrere Federn ausgenommen, können Spritzguß- oder KunststoffTeile sein. Der Spender ist mit einer Hand gleichzeitig sowohl zu tragen als auch hinsichtlich aller Funktionen zu betätigen.

Im Gehäuse sind zweckmäßig Leitungswege für das Medium vorgesehen, von denen mindestens einer hinsichtlich seiner Länge bzw. seines Durchlaßquerschnittes veränderbar ist. Hierzu kann im Gehäuse ein Kolben, ein Ventil oder ein anderer Steuerkörper vorgesehen sein. Bevorzugt umfaßt der Spender eine Pumpe, wie eine Schubkolbenpumpe, die ein oder mehrere manuell betätigbare Ventile bzw. ein oder mehrere Überdruckventile aufweist.

Besonders zweckmäßig ist es, wenn das Gehäuse mit einem Medienspeicher eine vormontierte Baueinheit so bildet, daß in Ausgangsstellung das Stechglied den Speicher noch nicht geöffnet hat. Erst durch manuelle Betätigung wird zuerst der Speicher geöffnet und anschließend das Medium zwischen dem Speicher und dem Gehäuse ausgetauscht, da beide dann miteinander kommunizieren.

Der Auslaß kann zur Abgabe von Tropfen, eines gebündelten Medienstrahles oder als Zerstäuberdüse ausgebildet sein. Vorteilhaft ist der Auslaß an einem Stutzen vorgesehen, der zur Einführung in eine Körperöffnung eines Patienten geeignet ist.

Das Gehäuse bzw. der für das Eindringen des Stechgliedes vorgesehene Körper können außen teilweise, zum größten Teil oder vollständig abgeschirmt sein, so daß ein Schutz vor Beschädigungen gegeben ist. Jeder Kolben des Spenders kann ein Ventil- und/oder Verdrängerkörper sein.

Vorteilhaft weist der Spender zwei quer zur Hubrichtung im Abstand voneinander liegende Handhaben auf, die einander bei der Betätigung angenähert werden und sowohl für den Stechhub als auch für einen Pumphub bzw. für einen Füllhub geeignet sind, durch welchen das Medium im Speicher in den Bereich des Medien-Einlasses gebracht wird. Jedem dieser Hübe kann im Bereich des Hubbeginnes eine Hemmung entgegenwirken, die durch erhöhte Betätigungskraft impulsartig überwindbar ist, wonach sofort die der weiteren Betätigung entgegenwirkenden Kräfte mehrfach niedriger als die Hemmkräfte sind. Dadurch können hohe Strömungsgeschwindigkeiten des Mediums bzw. eine hohe Auftreffgeschwindigkeit des Stechgliedes beim Einstechen erzielt werden.

Das Einstechen kann gegen eine Federkraft erfolgen, die unabhängig vom Einstichquerschnitt ist. Dadurch werden das Stechglied und der Einstichquerschnitt geschont, weil das Einstechen durch die Federkraft gedämpft und bei ausreichend großer Federspannung auch angetrieben wird. Zweckmäßig jedoch ist die Feder starr gehaltert und nur der zu durchstechende Körper elastisch.

Weitere Merkmale der Erfindung gehen auch aus der Beschreibung und den Zeichnungen hervor, wobei die einzelnen Merkmale jeweils für sich allein oder zu mehreren in Form von Unterkombinationen bei einer Ausführungsform der Erfindung verwirklicht sein und vorteilhafte Ausführungen darstellen können. Ausführungsbeispiele der Erfindung sind in den Zeichnungen dargestellt und werden im Folgenden näher erläutert. In den Zeichnungen zeigen:
- Fig. 1: einen erfindungsgemäßen Spender, teilweise im Axialschnitt,
- Fig. 2: eine weitere Ausführungsform in einer Darstellung gemäß Fig. 1 und
- Fig. 3: eine dritte Ausführungsform.

Der Spender 1 enthält ein Gehäuse 2, das als Medien-Durchtritte am einen Ende einen Medien-Einlaß 3 und am anderen Ende einen oder mehrere Medien-Auslässe 4, 4a aufweist. Das Gehäuse 2 wird mit Verbindungs-Mitteln 5 an einer Basis 6 mit einem Gehäuseteil starr befestigt. Die Basis 6 kann einen Hohlkörper 7, wie einen Medien-Speicher oder eine Ampulle bzw. Carpule umfassen oder nur durch diesen hohlen oder einen anderen Körper gebildet sein. Das Gehäuse 2 umfaßt eine Pumpe 8 oder Schubkolbenpumpe sowie eine Verschluß- oder Ventilanordnung 9 sowie ein Stechglied 10. Letzteres ist eine hohle Nadel aus Metall, deren freies Ende durch eine schräge oder ovale Stirnfläche eine scharfe Spitze bildet, die an den durchgehend zylindrischen Außenumfang anschließt. Der Einlaß 3 liegt daher spitzwinklig oder quer zur Nadelachse.

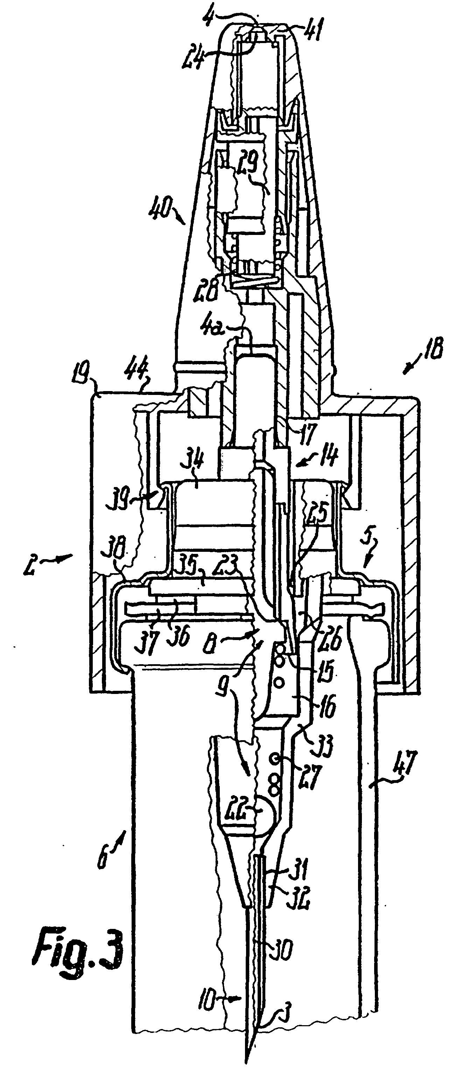

Der Spender 1 umfaßt drei in Längsrichtung aneinander gegliederte Einheiten 11 bis 13, von denen die zweite Einheit 12 gegenüber der ersten Einheit 11 und die dritte Einheit 13 gegenüber der zweiten Einheit 12 und mit dieser gegenüber der ersten Einheit 11 bewegbar, nämlich axial verschiebbar sind. Die zweite Einheit 12 trägt bewegbar oder axial verschiebbar den Speicher 7 sowie festsitzend den genannten Gehäuseteil des Gehäuses 2, der zur Einheit 12 gehört. Eine vorteilhafte Ausbildung der Pumpe 8 ist Fig. 3 im einzelnen zu entnehmen. Der genannte Gehäuseteil besteht aus einem einteiligen, längeren Hauptkörper 33 und einem einteiligen sowie kürzeren Deckel 34, wodurch ein Pumpen- oder Ventilgehäuse gebildet ist.

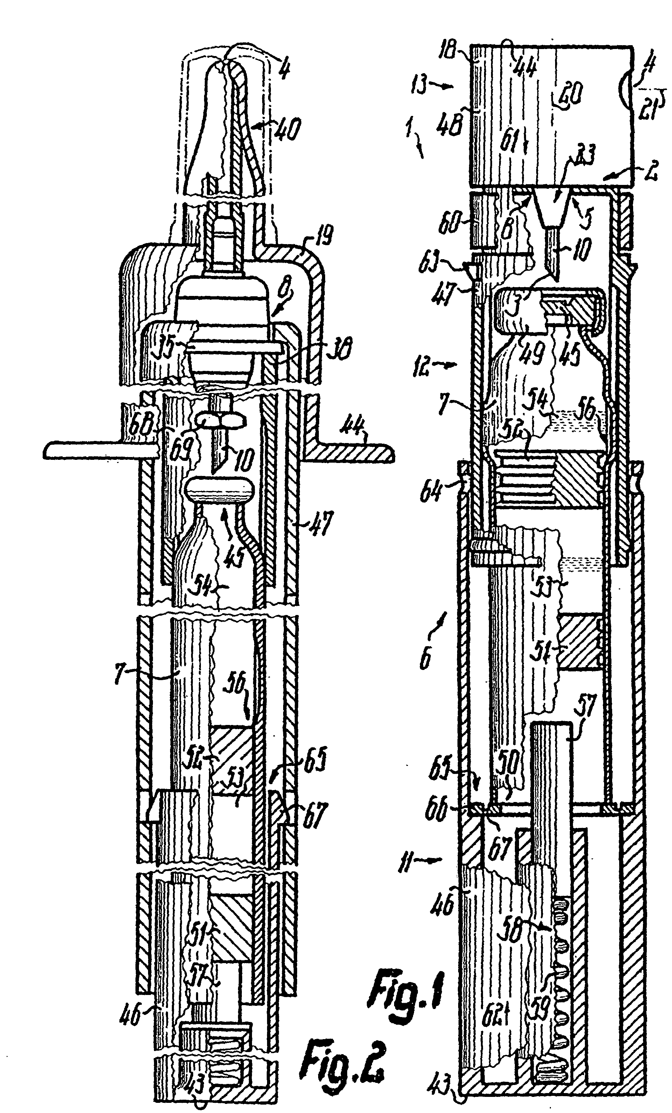

In diesem Gehäuse 33, 34 ist eine Kolbeneinheit 14 mit einem Kolben 15 axial verschiebbar, welcher abgedichtet am Innenumfang des Gehäuses gleitet und eine volumenvariable Kammer, wie eine Pumpkammer 16, begrenzt. Der an den Kolben 15 anschließende Stößel 17 der Einheit 14 ragt durch den Deckel 34 aus dem Gehäuse 33, 34 heraus und ist über eine Steckverbindung mit einem Kopf 18 festsitzend verbunden, der als Betätigungs- und Austragkopf dient. Die genannten Teile liegen in der Mittelachse 20 des Spenders 1, zu der die Achse 21 des ins Freie mündenden Auslasses 4 gemäß Fig. 1 quer oder rechtwinklig bzw. gemäß den Figuren 2 und 3 parallel oder achsgleich liegen kann. Dieser Auslaß 4 ist am Umfang des Kopfes 18 oder an dessen freiem Ende vorgesehen.

Die Ventilanordnung 9 umfaßt im Gehäuse 33, 34 ein EinlaßVentil 22, das zwischen der Kammer 16 und dem befestigten Ende der Kanüle oder Nadel 10 liegt. Außerdem liegt im Gehäuse 33, 34 ein Auslaß-Ventil 23, dessen beide Ventilkörper an der Einheit 14 angeordnet sind. Der bewegbare Ventilkörper ist durch den Innenumfang des hohlen Kolbens 15 gebildet, dessen Hülsenschaft die Ventilfeder zum Schließen des Ventiles bildet. Innerhalb des Gehäuses 33, 34 liegt auch ein Belüftungs-Ventil 25 einer Belüftung 26, um den Speicher zu belüften, falls dessen Speicherraum nicht volumenvariabel ist. Der bewegbare Ventilkörper des Ventiles 25 ist ebenfalls durch den Kolben 15 gebildet, während der Ventilsitz durch das Gehäuse bzw. dessen Deckel 34 gebildet ist. Die Luft kann von der Umgebung entlang des Stößels 17 in das Gehäuse 33, 34 und von dort durch eine Öffnung in der Gehäusewandung in den Speicher strömen.

Im Kopf 18 des längenvariablen Gehäuses 2 ist noch ein Auslaß-Ventil 24 vorgesehen, dessen Ventilkörper unmittelbar benachbart zum Auslaß 4 liegt. Die Einheit 14 wird zur Ausgangsstellung gemäß den Figuren 1 bis 3 mit einer Rückstellfeder 27 belastet, welche in der Kammer 16 liegt. In der Ausgangsstellung ist die Einheit 14 durch Anschlag bzw. Schließen des Ventiles 25 formschlüssig festgelegt, so daß mit Hubbeginn das Ventil 25 öffnet. Das Ventil 24 wird mit einer Feder 28 geschlossen. Jedes der Ventile 22 bis 25 kann ein Überdruckventil oder ein manuell durch Hub zu betätigendes Ventil sein sowie durch Anschlag seiner Ventilflächen schließen oder als Schieberventil ausgebildet sein. Die Einheit 14 bzw. der Stößel 17 ist ab dem Ventil 23 im Innern von einem Auslaß-Kanal 29 durchsetzt, dessen Ende den Auslaß bildet. Die Stößelverbindung mit dem Kopf 18 ist durch axiales Abziehen lösbar, wobei der Auslass 4a am Ende des Stößels 17 innerhalb des Kopfes 18 liegt.

Die Nadelbohrung des Gliedes 10 bildet einen an den Speicher 7 über die Öffnung 3 anzuschließenden Einlaß-Kanal 30, welcher unmittelbar gegen den Ventilkörper, wie eine Kugel, des Ventiles 22 und daher bei geöffnetem Ventil 22 unmittelbar in die Kammer 16 mündet. Das innere, kürzere Ende der Nadel 10 ist mit einer Befestigung 31 starr am Gehäuse 33 befestigt, nämlich von dessen innerem Endabschnitt 32 haftend umgeben und gegen Schubbelastungen durch formschlüssigen Anschlag seiner inneren Endfläche abgestützt. Der Hauptkörper 33 des Gehäuses ragt einschließlich der Nadel 10 über den größten Teil seiner Länge sowie am Außenumfang berührungsfrei in die Einheit 12.

Der Deckel 34 ist mit dem erweiterten Ende des Körpers 33 durch eine Schnappverbindung unlösbar verbunden und liegt mit dem größten Teil seiner Länge außerhalb der Einheit 12. Am Gehäuse oder Deckel 34 ist ein radial vorstehender, ringförmiger Flansch 35 vorgesehen, mit welchem das Gehäuse 33, 34 axial gegen eine Endfläche der Einheit 12 verspannt ist. Zwischen Flansch 35 und dieser Endfläche kann eine Dichtung 36 bzw. ein Filter 37 eingespannt sein. Auch durch das Filter 37 kann der Speicher 7, wie beschrieben, belüftet werden. Zur Verspannung ist ein Befestigungsglied, wie ein Krimpring oder eine Außenhülse der Einheit 12 vorgesehen. Der Kopf 18 ist gegen Abziehen von der Einheit 14 bzw. vom Gehäuse 33, 34 durch eine formschlüssige Sicherung 39 gesichert, welche innerhalb der Kappe 19 des Kopfes 18 bzw. vollständig innerhalb des Gehäuses 2 liegt. Gemäß den Figuren 2 und 3 weist der Kopf 18 einen frei vorstehenden Stutzen 40 zur Einführung in eine Körperöffnung, wie eine Nasenöffnung, auf. Das Ende des Stutzens 40 ist durch eine Stirnwand 41 gebildet, welche vom Kanal 29 bzw. Auslaß 4 durchsetzt ist. In Fig. 1 ist der Mantel der Kappe des Kopfes 18 in dieser Weise durchsetzt, wobei die Stirnwand der Kappe die Handhabe 44 bildet. Der Stutzen 40 steht einteilig von der Stirnwand der Kappe 19 vor, so daß die Handhabe 44 beiderseits des Stutzens 40 liegt. Die andere Handhabe 43 ist durch das von der Handhabe 44 abgekehrte Ende der Einheit 11 gebildet.

Der Spitze der Nadel 10 liegt mit geringem Abstand der Körper 7 bzw. eine von dessen Wandungen axial gegenüber. Diese Wandung ist ein Verschluß oder eine Membran 45, welche die Entleer-Öffnung des Speichers 7 dicht verschließt und mit einem ringförmigen Befestigungsglied, wie einem Krimpring 49, gesichert sowie verspannt ist. Die Membran 45 besteht aus druckelastischem Gummi von mehreren Millimetern Dicke und kann im zu durchstechenden Bereich in der Dicke reduziert sein. Die Membran 45 ist in einen verengten Hals des Speichers 7 eingesetzt und gegen eine Ringschulter im Innern dieses Halses gespannt. Die Befestigung 49 liegt dabei am Außenumfang berührungsfrei. Das vom Verschluß 45 abgekehrte Ende 50 des Speichers 7 ist auf einer Weite offen, die bis zum Hals konstant ist, so daß der Innenumfang eine Laufbahn für Verschlußglieder oder Kolben 51, 52 bildet.

Zwischen den beiden Kolben 51, 52 ist eine Kammer 53 und zwischen Kolben 52 und Membran 45 ein Medienraum 54 im Innenraum des Speichers 7 dicht begrenzt. Jede der Kammern 53, 54 kann mit einem der genannten Medien vollständig oder teilweise bzw. höchstens zur Hälfte gefüllt sein. Ein zu öffnender Verschluß oder ein Ventil 56 dient zur Leitungsverbindung der beiden Kammern 53, 54, z.B. so, daß das Medium aus der vom Verschluß 45 weiter entfernten Kammer 53 bei oben liegendem Auslaß 4 durch einen einzigen Hub in einer einzigen Richtung in die Kammer 54 umgefüllt werden kann. Das Ventil 56 ist ein Schieberventil, dessen verschiebbaren Ventilkörper der Kolben 52 bildet und das am Innenumfang des Speichers 7 Bypasskanäle zur Verbindung der Kammern 53, 54 aufweist.

Die freiliegenden Außenfläche der Einheiten 11 bis 13 sind durch hülsen- oder kappenförmige Einzelkörper 46 bis 48 gebildet, die teleskopartig gegeneinander verschiebbar sind. Gemäß Fig. 1 umgeben die Einzelkörper 46, 48 der Einheiten 11, 13 die voneinander abgekehrten Enden des Einzelkörpers 47 der Einheit 12, welche vollständig in den Einzelkörpern 46, 48 versenkt werden kann. Gemäß Fig. 2 greift der Einzelkörper 46 in das Innere des Einzelkörpers 12. Jeder der Einzelkörper 46 bis 48 ist einteilig ausgebildet. Die einander zugekehrten Umfangsflächen ineinandergreifender Einzelkörper sind gleitbar und abgedichtet oder mit geringen Spalten aneinander geführt. Der Körper 46 weist im Innern einen gegen den Kolben 51 gerichteten Stößel 57 auf, welcher in Ausgangsstellung gemäß Fig. 1 im Abstand vom Kolben 51 liegt und gemäß Fig. 2 am Kolben 51 anliegt. Der Stößel 57 ragt berührungsfrei in das Ende 50 hinein und ist in einer Gleitführung oder Hülse des Körpers 46 axial verschiebbar, nämlich gegen die Kraft einer Druckfeder 59. Diese gehört zu Federmitteln 58, welche auch die kompressiblen Gas- oder Luftfüllungen in den Kammern 53, 54 umfassen können.

Je nach den Erfordernissen wird mit einem ersten Hub zuerst die Membran 45 durchstochen und dann mit einem anschließenden, gleichgerichteten Hub von der Kammer 53 in die Kammer 54 umgefüllt oder es kann in besonderen Fällen zuerst die Umfüllung und dann das Durchstechen der Membran 45 erfolgen.

Gemäß Fig. 1 wird durch Drücken beider Handhaben 43, 44 zuerst die Einheit 11 unter Verkürzung des Spenders 1 in Richtung 62 verschoben, bis der Speicher 7 so weit mitgenommen ist, daß die Nadel 10 die Membran 45 durchstochen hat. Dann erst wird der Stößel 57 freigegeben bzw. relativ zum Speicher 7 bewegt, bis er am Kolben 51 anschlägt und diesen Kolben 51 mitnimmt. Bei vollständiger Füllung der Kammer 53 mit inkompressibler Flüssigkeit oder bei Vorhandensein eines entsprechenden Mitnehmers wird der Kolben 52 simultan mit dem Kolben 51 mitgenommen. Bei teilweise kompressibler Füllung der Kammer 53 oder anderer federnder Verbindung zwischen den Kolben bleibt der Kolben 52 zunächst stehen, bis eine ausreichend hohe Kompression oder Federspannung erreicht ist, wonach dann erst nach Verkleinerung der Kammer 53 auch der Kolben 52 mitgenommen wird. Dadurch kommt der Kolben 52 in eine Stellung, in welcher das Ventil 56 geöffnet ist und der Kolben 51 wird bis zum Anschlag zum Kolben 52 weiter geschoben, so daß das Medium aus der Kammer 53 vollständig in die Kammer 54 umgefüllt wird und sich mit dem dort vorhandenen Medium vermischt bzw. dieses in Lösung überführt.

Dieser Hub kann jetzt beendet sein oder noch weitergeführt werden, um das Medium in der Kammer 54 unter Druck zu setzen. Die Federmittel 58 dämpfen diesen Hub, wobei die Feder 59 vorgespannt wird und den Kolben 51 gegenüber dem Hub 62 verzögert antreibt, nämlich, wenn die Einheit 11 vor dem Kolben 51 die zugehörige Endstellung erreicht hat bzw. dem Kolben 51 vorauseilt. Dadurch wird die Umfüllungsgeschwindigkeit durch die Feder 59 vorgegeben und es werden zu hohe Drücke in der Kammer 54 vermieden. Am Ende dieses Hubes werden die Einheiten 11, 12 gegeneinander verriegelt, z.B. durch radial federnde Schnappglieder 63 des Körpers 47, die in Gegenglieder 64, wie Öffnungen, im Mantel des Körpers 46 automatisch einspringen und dann durch die Feder 59 spielfrei verspannt sind. Die Feder 39 kann auch den Kolben 51 nur so weit bewegen, bis er den Kolben 52 berührt, ohne den Kolben 2 dann mitzunehmen. Dadurch sowie durch die vollständige Entleerung der Kammer 53 wird auch das optimale Mischungsverhältnis zwischen den Medien erreicht. Die Feder 59 kann aber auch den Kolben 52 so weit in Richtung 62 bewegen, daß die Kammer 54 ein genau vorbestimmtes Volumen hat, das dann der mit dem Spender auszubringenden Nennfüllmenge entspricht. Bei dieser Bewegung wird die Luft vollständig aus der Kammer 54 herausgedrückt wie ggf. auch ein die Nennfüllmenge überschreitendes Volumen des nicht gasförmigen Mediums oder Gemisches. Hierzu kann der Weg des Stößels 57 in Richtung 62 unmittelbar durch Anschlag begrenzt sein, z.B. mit einem federnden Schnappnocken, der in eine Längsführung der Gleithülse eingreift. Die Feder 59 ist dann auch in dieser End- bzw. Anschlagstellung gespannt. Der Anschlag kann auch unmittelbar auf die Feder 59 wirken.

Während des Hubes 62 ist die Einheit 13 gegen Betätigung in entgegengesetzte Richtung 61 gesperrt, wofür zwischen dem offenen Ende der Kappe 18, 48 und dem Sperrglied 63 eine formschlüssige Sicherung oder Sperre vorgesehen ist. Deren Sperrglied 60 ist ein Ring oder eine Hülse, welche den Körper 47 eng umgibt und abgerissen oder radial abgezogen werden kann, so daß auch als Originalitätssicherung geeignet ist. Eine entsprechende Sperre 65 bewirkt die beschriebene Reihenfolge der Funktionen und setzt auch dem Hub 62 einen Widerstand entgegen. Gemäß Fig. 1 nimmt die sperre 65 zuerst den Speicher 7 mit, bis die Membran 45 durchstochen ist. Dann kann die Sperre 65 jedoch durch Aufbieten einer ausreichend hohen Betätigungskraft so überwunden werden, daß sofort danach bis zum Hubende ein wesentlich geringerer Widerstand gegen den Hub 62 gegeben ist. Das Sperrglied 66 der Sperre 65 ist ein Ring, welcher den Stößel 57 umgibt und mit Abstand zwischen seinem Außen- und Innenumfang eine geschwächte Zone oder Bruchstelle 67 aufweist. Der außerhalb der Stelle 67 liegende Ringteil ist gegen Bewegungen in Richtung 61 an einer Innenschulter des Körpers 46 abgestützt und der innere Ringteil ist gegen Bewegungen in Richtung 62 an der Endfläche des Speichers 7 abgestützt. Nach dem Membran-Öffnungsweg des Hubes 62, nach welchem die Nadel 10 relativ zur Membran 45 festgesetzt ist, reißt der innere Ringteil an der Stelle 67 vom äußeren Ringteil nach zunächst federnder Verformung ab und der Stößel 57 beginnt seinen Hub in Richtung 62.

Mit dem ersten Teil des Hubes 62 wird also vor Lösen der Sicherung 60 der Speicher 7 in Richtung 62 verschoben, wobei die Nadel 10 ohne Berührung des Gliedes 59 die Membran 45 durchsticht und dadurch der Einlaß 3 mit der Kammer 54 kommuniziert. Beim Verschieben ist der Speicher 7 am Innenumfang des Körpers 47 gleitbar geführt, wobei er eine reine Axial- oder auch eine drehende Schraubbewegung ausführen kann. Hierzu bildet die konstant dicke Wand des Speichers 7 im Bereich des Ventiles 56 radial vorstehende Nocken, die in axialen oder steilwendelförmigen Nuten am Innenumfang des Körpers 47 geführt sind.

Nach Lösen der Sperre 60 kann nunmehr der Körper 48 einschließlich Kolbeneinheit 14 gegenüber den Einheiten 11, 12, 33, 34 unter weiterer Verkürzung des Spenders 1 in Richtung 61 über einen Pump- bzw. Ventilöffnungs-Hub durch Betätigen derselben Handhaben 43, 44 bewegt werden, so daß die Kammer 16 verkleinert und entlüftet sowie danach bei dem durch die Feder 27 angetriebenen Rückhub durch den Kanal 30 und unter Öffnung des Ventiles 23 mit einem Teil des Mediums aus der Kammer 54 gefüllt wird. Beim nächsten Hub 61 wird das Medium in der Kammer 16 unter Druck gesetzt, wodurch das Ventil 22 schließt, während das Ventil 23 öffnet und das Medium durch den Kolben 15 und den Stößel 17 in das Innere des Kopfes 18 sowie bis zum Ventil 24 strömt, das unter dem Mediendruck ebenfalls öffnet, nämlich durch Bewegung des innen liegenden Ventilkörpers in Richtung 61. Die Öffnungsbewegung des Ventilkörpers des Ventiles 23 ist demgegenüber entgegengesetzt. Nach jedem Rückhub wird die Kammer 16 erneut aus der Kammer 54 gefüllt und mit jedem darauf folgenden Pumphub wird die so dosierte Medienmenge aus dem Auslaß 4 ausgetragen.

Gemäß Fig. 2 wird zuerst die Einheit 13 über den Hub 61 bewegt und dadurch die Kammer 16 verkleinert sowie entlüftet. Danach kann zuerst die beschriebene Umfüllung oder das Einstechen in den Verschluß 45 erfolgen. Zweckmäßig wird zunächst der Speicher 7 in Richtung 62 mitgenommen und der Verschluß 45 von der Nadel 10 durchstochen, wonach das Umfüllen in die Kammer 54 erfolgt. Dann liegt die Handhabe 43 in der Ebene des zugehörigen Endes des Körpers 47, in den der Körper 46 vollständig versenkt ist. Wird nun die Handhabe 44 freigegeben, so führt die Pumpe 8 den Rückhub aus und die Kammer 16 wird sofort mit Medium aus der Kammer 54 gefüllt. Durch das Öffnen der Membran 45 wird verhindert, daß beim Umfüllen in der Kammer 54 ein zu hoher Druck bzw. Überdruck herrscht und durch das Ventil 56 in die Kammer 53 übertragen wird.

Gemäß Fig. 2 ist das Befestigungsglied 38 durch ein gesondertes Glied 68, wie eine Hülse, gebildet, welche den Flansch 35 in Richtung 62 gegen eine innere Ringschulter am Ende des Körpers 47 spannt und in nicht näher dargestellter Weise gegenüber dem Körper 47 festgesetzt ist. Am Innenumfang der Hülse 68 ist der Speicher 7 verschiebbar geführt. Um die Einstechtiefe der Nadel 10 zu begrenzen, kann über deren Außenumfang ein Anschlag 69 vorstehen, welche an der äußeren Stirnfläche der Membran 45 am Ende des Stechhubes federnd anschlägt. Die Hemmung 65 ist hier durch Schnappglieder 67 des Körpers 46 gebildet, welche in Öffnungen im Mantel des Körpers 47 eingreifen und in der Ausgangsstellung die Körper 46, 47 gegen Auseinanderziehen formschlüssig sichern.

Alle Merkmale jeder Ausführungsform können auch bei jeder der übrigen Ausführungsformen vorgesehen sein, weshalb alle Beschreibungsteile sinngemäß für alle Ausführungsformen gelten. Die Merkmale und Wirkungen können genau oder nur im wesentlichen bzw. etwa, wie angegeben, vorgesehen sein und je nach den Erfordernissen auch stärker davon abweichen. Der Spender 1 ermöglicht eine hermetische Aufbewahrung des Mediums im Speicher 7 bis zum Gebrauch.

## Patentansprüche

1. Spender für Medien, mit einem vom Medium durchströmten Gehäuse (2), das gesonderte Medien-Durchtritte, nämlich einen Medien-Einlaß (3) und einen Medien-Auslaß (4, 4a) sowie Mittel (5) zur Verbindung mit einer Basis (6), in der ein Medienspeicher (7) vorgesehen ist, aufweist, wobei mindestens einer der Medien-Durchtritte von einem Stechglied (10), wie einer hohlen Nadel, begrenzt ist und wobei das Gehäuse (2) ein Ventil- bzw. Pumpengehäuse (33, 34) umfasst, daß der Medienspeicher (7) eine dem Stechglied (10) zugeordnete Ampulle, wie eine Carpule, und sein Innenraum an seinem von dem Stechglied (10) entfernten Ende (50) offen ist, **dadurch gekennzeichnet, daß** in dem Medienspeicher zwei Verschlußglieder (51, 52) bewegbar angeordnet sind, wobei ein Verschlußglied (52) zwei gesonderte Medienräume (53, 54) voneinander trennt, und ein Ventil (56) zur Verbindung der Medienräume (53, 54) vorgesehen ist.

2. Spender nach Anspruch 1, **dadurch gekennzeichnet, daß** die Kolbeneinheit (14) gegenüber dem Stechglied (10) bewegbar ist und im Pumpengehäuse (33, 34) eine Pumpkammer (16) begrenzt, daß das Ventil mindestens ein mit der Kolbeneinheit (14) verschiebbares Auslaß-Ventil (23, 24) ist und die Kolbeneinheit (14) vom Medium durchströmt ist.

3. Spender nach Anspruch 1 oder 2, **dadurch gekennzeichnet, daß** der Medien-Einlaß (3) von dem Stechglied (10) begrenzt ist, das mindestens eines der Merkmale aufweist:
a) es hat wenigstens eine Stechspitze,
b) es besteht aus Metall,
c) seine freiliegende Länge ist kürzer als das Gehäuse (2, 33, 34),
d) es ist unlösbar mit dem Gehäuse (2, 33, 34) verbunden,
e) es greift innen in das Gehäuse (2, 33, 34) ein,
f) es mündet gegen mindestens ein Auslaß-Ventil (22, 23, 24) im Gehäuse,
g) es mündet gegen eine im Gehäuse verschiebbare Kolbeneinheit (14), und
h) über seinen Umfang steht ein Anschlag (69) vor.

4. Spender nach einem der vorherigen Ansprüche, **dadurch gekennzeichnet, daß** der Medien-Auslaß (4) im Abstand vom Stechglied (10) eine Wand (41) durchsetzt, wobei die Wand (41) an einer Kappe (19) vorgesehen ist, welche eine Handhabe (44) aufweist, und wobei das Pumpengehäuse (33, 34) in die Kappe (19) hineinragt.

5. Spender nach einem der vorherigen Ansprüche, **dadurch gekennzeichnet, daß** dem Stechglied (10) eine Membran (45) der Basis (6) gegenüberliegt und relativ zu dieser Membran bis zur Durchdringung bewegbar ist, daß das Stechglied (10) und die Membran (45) an einem Grundkörper (47) der Basis (6) gelagert sind, und daß ein Hohlkörper (7, 68) der Basis (6) sowie das Pumpengehäuse (33, 34) vom Grundkörper (47) abgeschirmt sind.

6. Spender nach einem der vorherigen Ansprüche, **dadurch gekennzeichnet, daß** die Basis (6) zur Bewegung des Stechgliedes (10) zwei relativ zueinander über einen Hub (62) verschiebbare Einzelkörper (46, 47) umfaßt, von denen der erste Einzelkörper (46) den Medienspeicher (7) und der zweite Einzelkörper (47) das Pumpengehäuse (33, 34) abstützt, daß der Medienspeicher (7) am zweiten Einzelkörper (47) verschiebbar gelagert ist, und daß vorzugsweise mindestens eine dem Hub (62) entgegenwirkende Hemmung (65) vorgesehen ist, die nach Überwindung ihrer Schwergängigkeit eine wesentlich leichtere Gängigkeit hat.

7. Spender nach Anspruch 6, **dadurch gekennzeichnet, dass** zwischen den Einzelkörpern (46, 47) eine mit federnden Schnappgliedern (63) arbeitende, am Hubende wirksame Verriegelung vorgesehen ist.

8. Spender nach einem der vorherigen Ansprüche, **dadurch gekennzeichnet, daß** in den dem Stechglied (10) zugeordneten Innenraum ein Stößel (57) bewegbar ist, daß mit dem Stößel (57) das Volumen des Innenraumes variabel ist, und daß vorzugsweise der Stößel (57) am ersten Einzelkörper (46) angeordnet ist.

9. Spender nach einem der vorherigen Ansprüche, **dadurch gekennzeichnet, daß** Mittel (58) zur Federbelastung der Basis (6) bzw. des dem Stechglied (10) zugeordneten Verschlußgliedes (51, 52) vorgesehen sind, daß der Innenraum des Medienspeichers unter Vermittlung über eine Feder (59) volumenvariabel ist.

10. Spender nach Anspruch 8 oder 9, **dadurch gekennzeichnet, daß** der Stößel (57) federnd gelagert ist.

11. Spender nach einem der vorherigen Ansprüche, **gekennzeichnet durch** eine lösbare Sicherung (60) zwischen dem zweiten Einzelkörper (47) und einer das Ventil- bzw. Pumpengehäuse (39, 34) enthaltenden Einheit (13).

## Claims

1. A media dispenser including a body (2) communicating the flow of the medium and comprising separate medium passages namely a medium inlet (3) and a medium outlet (4, 4a) as well as means (5) for connecting to a base (6) in which a reservoir (7) is provided, at least one of the medium passages being defined by a piercing member (10), such as a hollow needle, the body comprising a valve or pump body (33, 34), the reservoir (7) being a medication cartridge, such as a carpule, assigned to the piercing member (10) and its interior space being open at its end (50) remote from the piercing member (10), **characterized in that** two closure members (51, 52) are movingly arranged in the medium reservoir, one closure member (52) separating two separate medium spaces (53, 54) from each other, and a valve (56) is provided for connecting the medium spaces (53, 54).

2. The dispenser as set forth in claim 1, **characterized in that** the piston unit (14) is movable relative to the piercing member (10) and defines in the pump body (33, 34) a pumping chamber (16), that the valve is at least one outlet valve (23, 24) shiftable with the piston unit (14) and that the piston unit (14) communicates the medium.

3. The dispenser as set forth in claim 1 or 2, **characterized in that** the medium inlet (3) is defined by the piercing member (10) comprising at least one of the features;
a) it including at least one piercing tip,
b) it being made of metal,
c) its exposed length being shorter than the body (2,33,34),
d) it being non-releasably connected to the body (2,33,34),
e) it engaging internally the body (2,33,34),
f) it porting against at least one outlet valve (22, 23, 24) in the body,
g) it porting against a piston unit (14) shiftingly located in the body, and
h) a stop (69) protruding beyond its circumference.

4. The dispenser as set forth in any of the preceding claims, **characterized in that** the medium outlet (4) passes through a wall (41) spaced away from the piercing member (10), the wall (41) being provided at a cap (19) comprising a finger rest (44), and the pump body (33, 34) protruding into the cap (19).

5. The dispenser as set forth in any of the preceding claims, **characterized in that** a diaphragm (45) of the base (6) is located opposite the piercing member (10) which is movable relative to the diaphragm up to being pierced, the piercing member (10) and the diaphragm (45) being mounted on a basic element (47) of the base (6), and that a hollow body (7, 68) of the base (6) as well as the pump body (33, 34) are shielded from the basic element (47).

6. The dispenser as set forth in any of the preceding claims, **characterized in that** for moving the piercing member (10) the base (6) comprises two single elements (46, 47) mutually shiftable over a stroke (62), of which the first single element (46) supports the reservoir (7) and the second single element (47) supports the pump body (33, 34), that the reservoir (7) is shiftingly located on the second single element (47), and that preferably at least one restraint (65) opposing the stroke (62) is provided which has substantially greater freedom of movement once its restraining action is defeated.

7. The dispenser as set forth in claim 6, **characterized in that** a latch working with spring-loaded snap-action members (63) and effective at the end of the stroke is provided between the single elements (46, 47).

8. The dispenser as set forth in any of the preceding claims, **characterized in that** a plunger (57) is movable into the interior space assigned the piercing member (10), the volume of the interior space being variable by means of the plunger (57), and that preferably the plunger (57) is arranged on the first single element (46).

9. The dispenser as set forth in any of the preceding claims, **characterized in that** means (58) for spring-loading the base (6) or the closure member (51, 52) assigned the piercing member (10) are provided, that the volume of the interior space of the medium reservoir is variable with the aid of a spring (59).

10. The dispenser as set forth in claim 8 or 9, **characterized in that** the plunger (57) is spring-loaded.

11. The dispenser as set forth in any of the preceding claims, **characterized by** a releasable lock (60) between the second single element (47) and a unit (13) containing the valve or pump body (39, 34).

## Revendications

1. Distributeur de substances, avec un boîtier (2), qui est traversé par le courant de substance et qui présente des passages à substance individuels, c'est-à-dire une entrée de substance (3) et une sortie de substance (4, 4a) ainsi que des moyens (5) pour le raccordement avec une base (6), dans laquelle est prévu le réservoir à substances (7), où au moins un des passages à substance est délimité par un élément de perçage (10), tel qu'une aiguille creuse, où le boîtier (2) comprend un boîtier de soupape ou encore de pompe (33, 34), et où le réservoir à substances (7) est une ampoule, associée à l'élément de perçage (10), telle qu'une carpoule et son espace intérieur est ouvert à son extrémité (50) éloignée de l'élément de perçage (10), **caractérisé en ce que** deux éléments de fermeture (51, 52) sont disposés de manière mobile dans le réservoir à substances, un élément de fermeture (52) séparant mutuellement deux espaces à substance (53, 54), et une soupape (56) étant prévue pour le raccordement des espaces à substance (53, 54).

2. Distributeur d'après la revendication 1, **caractérisé en ce que** l'unité de piston (14) est déplaçable par rapport à l'élément de perçage (10) et qu'elle délimite une chambre de pompage (16) dans le boîtier de pompe (33, 34), **en ce que** la soupape est constituée au moins d'une soupape de sortie (23, 24) déplaçable avec l'unité de piston (14) et que l'unité de piston (14) est traversée par l'écoulement de substance.

3. Distributeur d'après la revendication 1 ou 2, **caractérisé en ce que** l'entrée de substance (3) est délimitée par l'élément de perçage (10), qui présente au moins une des caractéristiques suivantes :
a) il présente au moins une pointe de perçage,
b) il est composé de métal,
c) sa longueur à découvert est plus courte que le boîtier (2, 33, 34),
d) il est raccordé au boîtier (2, 33, 34) de manière indesserrable,
e) il s'engage à l'intérieur du boîtier (2, 33, 34),
f) il débouche contre au moins une soupape de sortie (22, 23, 24) dans le boîtier,
g) il débouche contre une unité de piston (14) déplaçable dans le boîtier, et
h) un arrêt (69) saille au-delà de son périmètre.

4. Distributeur d'après une des revendications précédentes, **caractérisé en ce que** la sortie de substances (4) traverse une paroi (41) à une certaine distance de l'élément de perçage (10), la paroi (41) étant prévue sur un capuchon (19), qui présente un élément de préhension (44), le boîtier de pompe (33, 34) saillant à l'intérieur du capuchon (19).

5. Distributeur d'après une des revendications précédentes, **caractérisé en ce que** en face de l'élément de perçage (10) se trouve une membrane (45) de la base (6) et que, jusqu'à la pénétration, cet élément de perçage est déplaçable par rapport à cette membrane, **en ce que** l'élément de perçage (10) et la membrane (45) sont logés sur un corps de base (47) de la base (6), et **en ce qu'**un corps creux (7, 68) de la base (6) ainsi que le boîtier de pompe (33, 34) sont écrannés par rapport au corps de base (47).

6. Distributeur d'après une des revendications précédentes, **caractérisé en ce que** pour le déplacement de l'élément de perçage (10), la base (6) comprend deux corps individuels (46, 47) mobiles l'un par rapport à l'autre au moyen d'une course (62), dont le premier corps individuel (46) soutient le réservoir à substances (7) et le deuxième corps individuel (47) soutient le boîtier de pompe (33, 34), **en ce que** le réservoir à substances (7) est logé de manière déplaçable auprès du deuxième corps individuel (47), et **en ce qu'**on prévoit de préférence au moins un moyen d'inhibition (65) agissant contre le mouvement de course (62), qui présente une beaucoup plus grande souplesse de mouvement après avoir surmonté une certaine résistance au fonctionnement.

7. Distributeur d'après la revendication 6, **caractérisé en ce qu'**entre les corps individuels (46, 47) on prévoit un mécanisme de verrouillage opérant au moyen d'éléments à déclic (63) et étant actif à la fin de la course.

8. Distributeur d'après une des revendications précédentes, **caractérisé en ce que** dans l'espace intérieur associé à l'élément de perçage (10) un poussoir (57) est déplaçable, **en ce que** le volume de l'espace intérieur peut être varié au moyen du poussoir (57), et **en ce que** de préférence le poussoir (57) est disposé sur le premier corps individuel (46).

9. Distributeur d'après une des revendications précédentes, **caractérisé en ce qu'**on prévoit des moyen (58) pour une charge obtenue sous l'action d'un ressort de la base (6) ou encore de l'élément de fermeture (51, 52) associé à l'élément de perçage (10), **en ce que** l'espace intérieur du réservoir à substances peut être varié dans son volume au moyen d'un ressort (59).

10. Distributeur d'après la revendication 8 ou 9, **caractérisé en ce que** le poussoir (57) est logé de manière élastique.

11. Distributeur d'après une des revendications précédentes, **caractérisé par** un assurage (60) amovible entre le deuxième corps individuel (47) et une unité (13) qui comprend le boîtier de soupape ou encore de pompe (39, 34).
